# EUROPEAN PATENT APPLICATION

(11) **EP 4 494 644 A1**
(43) Date of publication of application: **22.01.2025**
(21) Application number: 23770776.5
(22) Date of filing: 14.03.2023
(51) Int. Cl.: A61K 31/7088, A61K 47/18, A61K 47/24, A61K 47/28, A61K 47/34, A61K 48/00, C12N 15/88

(54) **LIPID COMPOSITION**

(30) Priority: 15.03.2022 JP 2022039874
(71) Applicant: FUJIFILM Corporation, Tokyo 106-8620 (JP)
(72) Inventor: KASAGI Noriyuki, Ashigarakami-gun, Kanagawa 258-8577 (JP); YOSHIMURA Masashi, Ashigarakami-gun, Kanagawa 258-8577 (JP); MAKITA Keiko, Ashigarakami-gun, Kanagawa 258-8577 (JP); TANABE Shintaro, Ashigarakami-gun, Kanagawa 258-8577 (JP); NITABARU Tatsuya, Ashigarakami-gun, Kanagawa 258-8577 (JP)
(74) Representative: Klunker IP Patentanwälte PartG mbB
(86) International application number: PCT/JP2023/009821
(87) International publication number: WO 2023/176821

(57) **Abstract**

An object of the present invention is to provide a lipid composition capable of realizing an excellent nucleic acid delivery efficiency. According to the present invention, there is provided a lipid composition containing a compound represented by Formula (1), a lipid represented by Formula (2) or a salt of the lipid, a neutral lipid, a nonionic hydrophilic polymer, and a lipid having a nucleic acid.

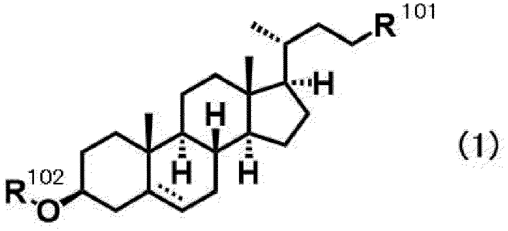

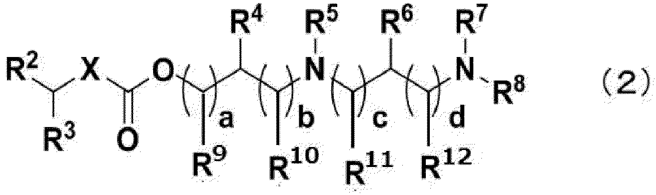

The definition of each symbol in the formulae is as described in the present specification.

## Description

### Technical Field

The present invention relates to a lipid composition containing a cholesterol derivative and a lipid.

### Background Art

Nucleic acid drugs have a clear mechanism of action on diseases, have few side effects, and are expected as next-generation drugs, and nucleic acid drugs are being actively developed. As one technique for delivering a nucleic acid to a cell, a method of encapsulating the nucleic acid in a liposome or a lipid particle and administering the nucleic acid is known. In this technique, as the lipids, lipids having a substituent such as an amino group, which turn into cations at a low pH, are used and appropriate charge is applied to the particles to realize the delivery of nucleic acids.

As a compound to be incorporated into the lipid particles, Patent Documents 1 and 2 disclose a compound having an ester group, an acetal group, or the like as a linking group that links an aliphatic group to an amino group. Patent Document 3 discloses a compound having a vinyloxy group, an amide group, an oxime group, or the like as a linking group that links an aliphatic group to an amino group. Patent Document 4 describes lipid nanoparticles containing DLin-MC3-DMA that is a cationic lipid. Patent Document 5 describes cationic lipids that are useful for delivering a drug to cells and tissues. In Patent Document 6, there are studies on a type and a composition ratio of a lipid compound to be combined in a case of producing nucleic acid-containing particles.

Examples of a major method for enhancing the drug efficacy of the nucleic acid drug include a method of improving cellular uptake and a method of improving an endosome escape efficiency. Study on improvement of cellular uptake has been actively conducted mainly on the development of ionizable lipids, but there is almost no study on improvement of the endosome escape efficiency. The lipid particle encapsulating a nucleic acid is generally composed of an ionizable lipid/helper lipid/cholesterol/polyethylene glycol lipid. In Non-Patent Document 1, it has been reported that the endosome escape efficiency is improved by focusing on cholesterol. In addition, in Patent Document 7, lipid nanoparticles (LNP) that enable delivery of a nucleic acid to an immune cell, particularly a T cell or a dendritic cell has been reported.

### Prior Art Documents

### Patent Documents

Patent Document 1: WO2010/054401A
Patent Document 2: WO2010/144740A
Patent Document 3: WO2010/054405A
Patent Document 4: US2013-0245107A
Patent Document 5: WO2015/095340A
Patent Document 6: WO2009/127060A
Patent Document 7: WO2019/152557A

### Non-Patent Documents

Non-Patent Document 1: NATURE COMMUNICATIONS (2020) 11: 983

### SUMMARY OF THE INVENTION

### Object to be solved by the invention

There is still a demand for the development of a lipid composition capable of realizing an excellent nucleic acid delivery efficiency. An object of the present invention is to provide a lipid composition capable of realizing an excellent nucleic acid delivery efficiency.

### Means for solving the object

As a result of intensive studies to achieve the above-described object, the present inventors have found that, by using a compound represented by Formula (1) as the cholesterol derivative, and using a lipid represented by Formula (2) or a salt of the lipid, a neutral lipid, and a lipid having a nonionic hydrophilic polymer as the lipid, a lipid composition exhibiting an excellent nucleic acid delivery efficiency can be obtained. The present invention has been completed based on the above findings. According to the present invention, the following inventions are provided.
<1> A lipid composition containing a compound represented by Formula (1), a lipid represented by Formula (2) or a salt of the lipid, a neutral lipid, a lipid having a nonionic hydrophilic polymer, and a nucleic acid, 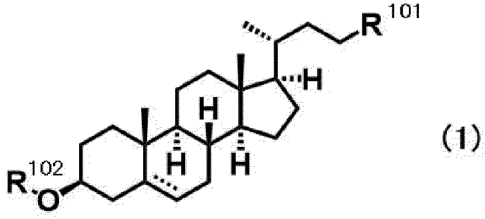 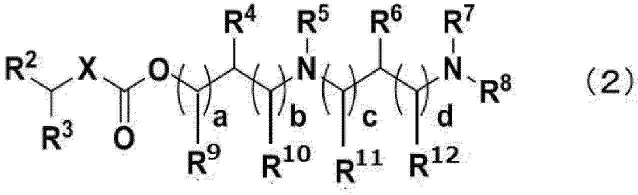 in Formula (1),
   R¹⁰¹ represents 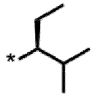 or 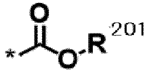
   , * represents a bonding position, R²⁰¹ represents a chain-like hydrocarbon group having 7 to 18 carbon atoms, 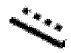
   represents a single bond or a double bond,
   R¹⁰² represents
      (i) a hydrogen atom,
      (ii) a hydrocarbon group having 1 to 4 carbon atoms, which may be substituted with one or more substituents selected from OH, SH, COOH, NR¹⁰⁶R¹⁰⁷, N(R¹⁰⁸)C(O)R¹⁰⁹, or CF₃,
      (iii) C(O)R¹⁰³, or
      (iv) C(O)NR¹⁰⁴R¹⁰⁵,
   R¹⁰³ represents a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
   R¹⁰⁴ and R¹⁰⁵ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
   R^{106,} R¹⁰⁷, R¹⁰⁸, and R¹⁰⁹ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃, and
   R¹¹⁰, R¹¹¹, R¹¹², and R¹¹³ each independently represent a hydrogen atom or a hydrocarbon group having 1 to 4 carbon atoms, which may be substituted with one or more substituents selected from OH or SH,
   in Formula (2), X represents -NR¹- or -O-,
   R¹ represents a hydrogen atom, a hydrocarbon group having 6 to 24 carbon atoms, or a group represented by R²¹-L¹-R^{22_}, where R²¹ represents a hydrocarbon group having 1 to 24 carbon atoms, L¹ represents -O(CO)O-, -O(CO)-, -(CO)O-, -O-, or 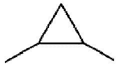
   , and R²² represents a divalent hydrocarbon linking group having 1 to 18 carbon atoms,
   R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 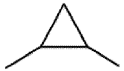
   , and R³² represents a divalent hydrocarbon linking group having 1 to 18 carbon atoms,
   R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
   in any one or more pairs of groups among R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸, the groups may be linked to each other to form a 4- to 7-membered ring which may contain an O atom,
   a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
   a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by - O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
   a, b, c, and d each independently represent an integer of 0 to 3, provided that a + b is 1 or more, and c + d is 1 or more.
<2> The lipid composition according to <1>, in which in Formula (1), R¹⁰² represents
   (i) a hydrogen atom, or
   (iv) C(O)NR¹⁰⁴R¹⁰⁵,

   R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³, and
   R¹¹⁰, R¹¹¹, R¹¹², and R¹¹³ each independently represent a hydrogen atom or a hydrocarbon group having 1 to 4 carbon atoms, which may be substituted with one or more OH's.
<3> The lipid composition according to <1>, in which R¹⁰¹ in Formula (1) represents 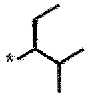 , and
   R¹⁰² represents C(O)NR¹⁰⁴R¹⁰⁵,
   R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³,
   R¹¹⁰, R¹¹¹, R¹¹², and R¹¹³ each independently represent a hydrogen atom or a hydrocarbon group having 1 to 4 carbon atoms, which may be substituted with one or more OH's.
<4> The lipid composition according to <1>, in which R¹⁰¹ in Formula (1) represents 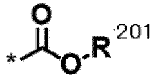 , R¹⁰² represents a hydrogen atom, and R²⁰¹ represents a chain-like hydrocarbon group having 7 to 13 carbon atoms.
<5> The lipid composition according to any one of <1> to <4>, in which the compound represented by Formula (1) is a compound represented by any of (A) to (M). 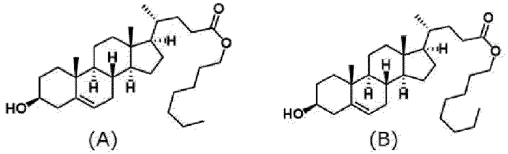 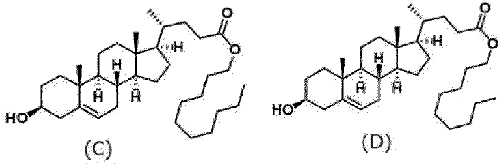 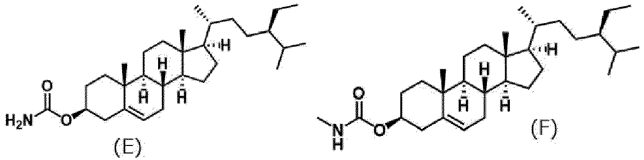 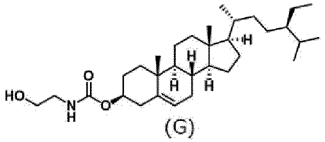 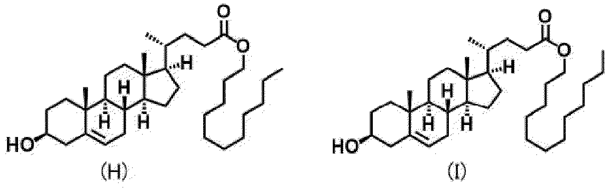 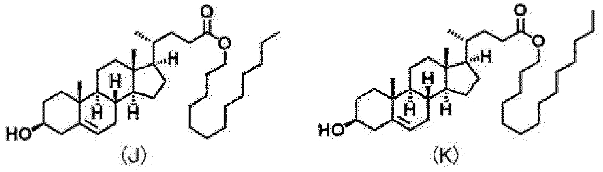 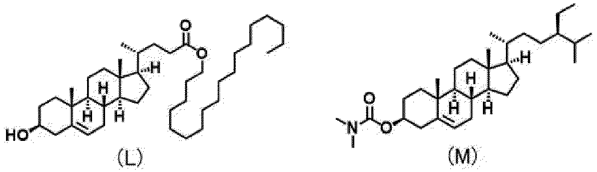
<6> The lipid composition according to any one of <1> to <5>, in which a content of the compound represented by Formula (1) is 10 mol% to 60 mol% with respect to total lipids.
<7> The lipid composition according to any one of <1> to <6>, in which a content of the lipid represented by Formula (2) or the salt of the lipid is 30 mol% to 70 mol% with respect to total lipids.
<8> The lipid composition according to any one of <1> to <7>, in which the neutral lipid is a zwitterionic lipid.
<9> The lipid composition according to any one of <1> to <8>, in which the neutral lipid is a phospholipid.
<10> The lipid composition according to any one of <1> to <9>, in which a content of the neutral lipid is 1 mol% to 30 mol% with respect to total lipids.
<11> The lipid composition according to any one of <1> to <10>, in which the lipid having the nonionic hydrophilic polymer contains an acyl group, and a carbon chain length of the acyl group is 8 to 26.
<12> The lipid composition according to any one of <1> to <11>, in which a content of the lipid having the nonionic hydrophilic polymer is 0.1 mol% to 10 mol% with respect to total lipids.
<13> The lipid composition according to any one of <1> to <12>, in which a weight ratio of lipids to the nucleic acid is 5 to 100.
<14> The lipid composition according to any one of <1> to <13>, in which the lipid composition is a composition for introducing a nucleic acid into a cell.
<15> The lipid composition according to any one of <1> to <13>, in which the lipid composition is a composition for nucleic acid delivery in vivo.
<16> The lipid composition according to any one of <1> to <15>, in which the lipid composition is a lipid particle.

### Effect of the invention

The lipid composition according to the embodiment of the present invention can realize an excellent nucleic acid delivery efficiency.

### EMBODIMENTS FOR CARRYING OUT THE INVENTION

Hereinafter, the present invention is specifically described.

In the present specification, "to" shows a range including numerical values described before and after "to" as a minimum value and a maximum value, respectively.

The lipid composition according to the embodiment of the present invention contains a compound represented by Formula (1), a lipid represented by Formula (2) or a salt of the lipid, a neutral lipid, a lipid having a nonionic hydrophilic polymer, and a nucleic acid. By adopting the above-described configuration, it has been found for the first time in the present invention that the effect of introducing the nucleic acid into a cell can be efficiently exhibited, that is, an excellent nucleic acid delivery efficiency can be realized.

### <Compound represented by Formula (1)>

In the present invention, a compound represented by Formula (1) is used.

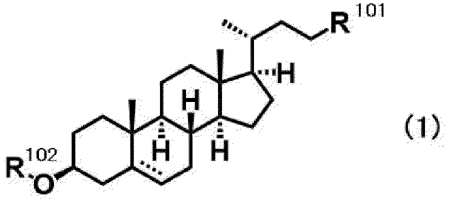

In Formula (1),
R¹⁰¹ represents 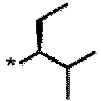 or 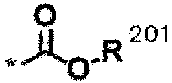
,* represents a bonding position, R²⁰¹ represents a chain-like hydrocarbon group having 7 to 18 carbon atoms, 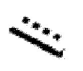
represents a single bond or a double bond,
R¹⁰² represents
   (i) a hydrogen atom,
   (ii) a hydrocarbon group having 1 to 4 carbon atoms, which may be substituted with one or more substituents selected from OH, SH, COOH, NR¹⁰⁶R¹⁰⁷, N(R¹⁰⁸)C(O)R¹⁰⁹, or CF₃,
   (iii) C(O)R¹⁰³, or
   (iv) C(O)NR¹⁰⁴R¹⁰⁵,
R¹⁰³ represents a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
R¹⁰⁴ and R¹⁰⁵ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
R¹⁰⁶, R¹⁰⁷, R¹⁰⁸, and R¹⁰⁹ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃, and
R¹¹⁰, R¹¹¹, R¹¹², and R¹¹³ each independently represent a hydrogen atom or a hydrocarbon group having 1 to 4 carbon atoms, which may be substituted with one or more substituents selected from OH or SH.

The chain-like hydrocarbon group having 7 to 18 carbon atoms in the definition of Formula (1) may be either a saturated hydrocarbon group or an unsaturated hydrocarbon group, and may be either linear or branched, but is preferably a saturated hydrocarbon group.

The hydrocarbon group having 1 to 4 carbon atoms in the definition of Formula (1) may be either a saturated hydrocarbon group or an unsaturated hydrocarbon group, and may be either linear or branched, but is preferably a saturated hydrocarbon group. Examples of the hydrocarbon group having 1 to 4 carbon atoms include a methyl group, an ethyl group, an n-propyl group, an isopropyl group, an n-butyl group, a sec-butyl group, an isobutyl group, a tert-butyl group, an ethylene group, an n-propylene group, an isopropylene group, an n-butylene group, an isobutylene group, and the like.

Preferably, in Formula (1), R¹⁰² represents
(i) a hydrogen atom, or
(iv) C(O)NR¹⁰⁴R¹⁰⁵,

R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³, and
R¹¹⁰, R¹¹¹, R¹¹², and R¹¹³ each independently represent a hydrogen atom or a hydrocarbon group having 1 to 4 carbon atoms, which may be substituted with one or more OH's.

Preferably, R¹⁰¹ in Formula (1) represents 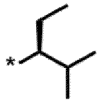 , and
R¹⁰² represents C(O)NR¹⁰⁴R¹⁰⁵,
R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³,
R¹¹⁰, R¹¹¹, R¹¹², and R¹¹³ each independently represent a hydrogen atom or a hydrocarbon group having 1 to 4 carbon atoms, which may be substituted with one or more OH's.

Preferably, R¹⁰¹ in Formula (1) represents 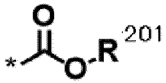 , R¹⁰² represents a hydrogen atom, and R²⁰¹ represents a chain-like hydrocarbon group having 7 to 13 carbon atoms.

Preferably, 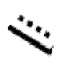 represents a double bond.

Particularly preferably, the compound represented by Formula (1) is a compound represented by any of (A) to (M).

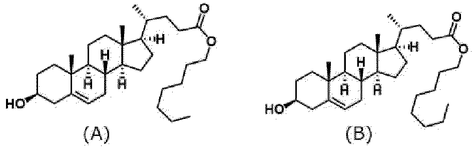

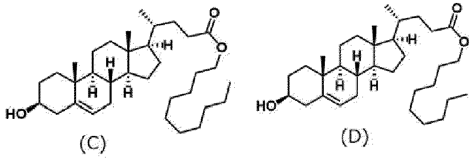

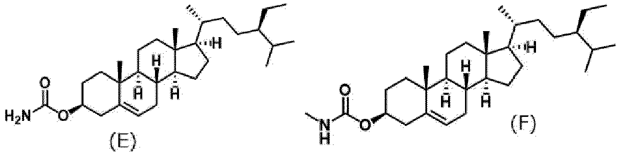

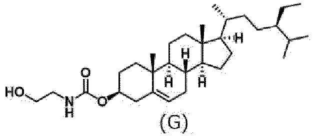

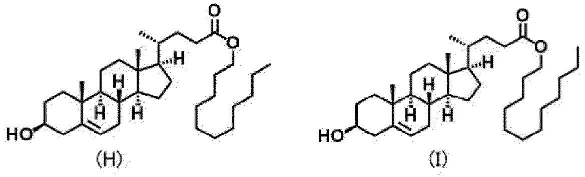

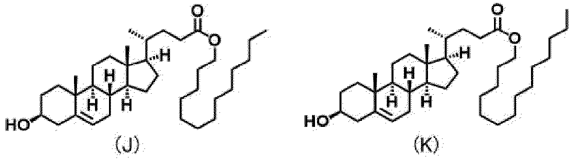

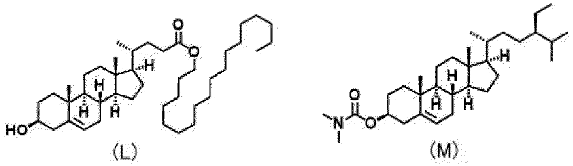

The CAS registration numbers of the compounds (A) to (G) are as follows.
Compound (A): CAS 331245-55-7
Compound (B): CAS 331245-51-3
Compound (C): CAS 331245-57-9
Compound (D): CAS 331245-56-8
Compound (E): CAS 104082-35-1
Compound (F): CAS 92588-73-3
Compound (G): CAS 136099-14-4

Among these, the compounds (B), (C), (D), and (G) are more preferable, and the compounds (D) and (G) are particularly preferable.

The compound represented by Formula (1) can be synthesized according to the method described in the synthesis of the compound in Examples which will be described later.

The content of the compound represented by Formula (1) is preferably 10 to 80 mol%, more preferably 10 mol% to 60 mol%, and still more preferably 30 mol% to 50 mol% with respect to the total lipids.

### <Lipid represented by Formula (2) or salt of lipid>

In the present invention, a lipid represented by Formula (2) or a salt of the lipid is used.

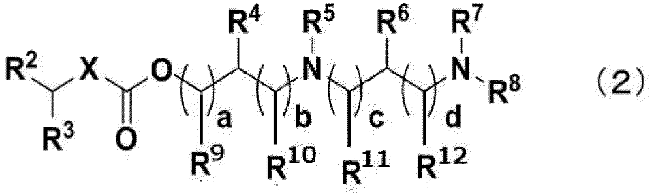

In the formula,
X represents -NR¹- or -O-,
R¹ represents a hydrogen atom, a hydrocarbon group having 6 to 24 carbon atoms, or a group represented by R²¹-L¹-R^{22_}, where R²¹ represents a hydrocarbon group having 1 to 24 carbon atoms, L¹ represents -O(CO)O-, -O(CO)-, -(CO)O-, -O-, or 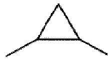
, and R²² represents a divalent hydrocarbon linking group having 1 to 18 carbon atoms,
Rand R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 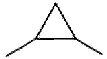
, and R³² represents a divalent hydrocarbon linking group having 1 to 18 carbon atoms,
R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
in any one or more pairs of groups among R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸, the groups may be linked to each other to form a 4- to 7-membered ring which may contain an O atom,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by - O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more.

As the hydrocarbon group having 6 to 24 carbon atoms that is represented by R¹ and the hydrocarbon group having 3 to 24 carbon atoms that is represented by R² and R³, an alkyl group, an alkenyl group, or an alkynyl group is preferable, and an alkyl group or an alkenyl group is more preferable. The alkyl group having 6 to 24 carbon atoms and the alkyl group having 3 to 24 carbon atoms may be linear or branched or may be chain-like or cyclic. The alkyl group having 6 to 24 carbon atoms is preferably an alkyl group having 6 to 20 carbon atoms, and the alkyl group having 3 to 24 carbon atoms is more preferably an alkyl group having 6 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, and an icosyl group. The alkenyl group having 6 to 24 carbon atoms and the alkenyl group having 3 to 24 carbon atoms may be linear or branched or may be chain-like or cyclic. The alkenyl group having 6 to 24 carbon atoms is preferably an alkenyl group having 6 to 20 carbon atoms, and the alkenyl group having 3 to 24 carbon atoms is more preferably an alkenyl group having 6 to 20 carbon atoms. Specifically, examples thereof include a hexenyl group, a heptenyl group, an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably an (8Z, 11Z)-heptadeca-8, 11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), and an icosadienyl group (preferably an (11,14)-icosa-11,14-dienyl group). The alkynyl group having 6 to 24 carbon atoms is preferably an alkynyl group having 6 to 20 carbon atoms, and the alkynyl group having 3 to 24 carbon atoms is more preferably an alkynyl group having 6 to 20 carbon atoms. Specifically, examples thereof include a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As hydrocarbon group having 1 to 24 carbon atoms that is represented by R²¹ and R³¹, an alkyl group having 10 to 24 carbon atoms, an alkenyl group having 10 to 24 carbon atoms, or an alkynyl group having 10 to 24 carbon atoms is preferable. The alkyl group having 10 to 24 carbon atoms may be linear or branched or may be chain-like or cyclic. The alkyl group having 10 to 24 carbon atoms is preferably an alkyl group having 12 to 24 carbon atoms. Specifically, examples thereof include a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, and a 2-(4,4-dimethylpentan-2-yl)-5,7,7-trimethyloctyl group. The alkenyl group having 10 to 24 carbon atoms may be linear or branched or may be chain-like or cyclic. Specifically, examples thereof include a decenyl group, an undecenyl group, a dodecenyl group, a dodecadienyl group, tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 10 to 24 carbon atoms may be linear or branched or may be chain-like or cyclic. Specifically, examples thereof include a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As the divalent hydrocarbon linking group having 1 to 18 carbon atoms that is represented by R²² and R³², an alkylene group having 1 to 18 carbon atoms or an alkenylene group having 2 to 18 carbon atoms is preferable. The alkylene group having 1 to 18 carbon atoms may be linear or branched or may be chain-like or cyclic. The number of carbon atoms in the alkylene group having 1 to 18 carbon atoms is preferably 1 to 12, more preferably 1 to 10, and still more preferably 2 to 10. Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and a dodecamethylene group. The alkenylene group having 2 to 18 carbon atoms may be linear or branched or may be chain-like or cyclic. The number of carbon atoms in the alkenylene group having 2 to 18 carbon atoms is preferably 1 to 12, and more preferably 2 to 10.

As a preferred range of L¹, -O(CO)O-, -O(CO)-, or -(CO)O- is preferable and -O(CO)- or -(CO)O- is more preferable.

As a preferred range of L², -O(CO)O-, -O(CO)-, or -(CO)O- is preferable and -O(CO)- or -(CO)O- is more preferable.

The alkyl group having 1 to 18 carbon atoms in the alkyl group having 1 to 18 carbon atoms which may be substituted and which is represented by R⁴, R⁶, R⁹, R¹⁰, R¹¹, and R¹² may be linear or branched or may be chain-like or cyclic. The number of carbon atoms in the alkyl group having 1 to 18 carbon atoms is preferably 1 to 12. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-R⁴¹, -O(CO)-R⁴², -(CO)O-R⁴³, or -O-R⁴⁴ is preferable, and a group represented by -O(CO)-R⁴² or -(CO)O-R⁴³ is more preferable.

The alkyl group having 1 to 18 carbon atoms in the alkyl group having 1 to 18 carbon atoms which may be substituted and which is represented by R⁵, R⁷, and R⁸ may be linear or branched or may be chain-like or cyclic. The number of carbon atoms in the alkyl group having 1 to 18 carbon atoms is preferably 1 to 12 and more preferably 1 to 8. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-R⁴¹, -O(CO)-R⁴², -(CO)O-R⁴³, or -O-R⁴⁴ is preferable, and a group represented by -O(CO)-R⁴² or -(CO)O-R⁴³ is more preferable.

Examples of the 4- to 7-membered ring which may contain an O atom include an azetidine ring, a pyrrolidine ring, a piperidine ring, a morpholine ring, and an azepane ring. The 4- to 7-membered ring is preferably a 6-membered ring and is preferably a piperidine ring or a morpholine ring.

In a case where the alkyl group having 1 to 18 carbon atoms which is represented by R⁴, R⁵, R⁶, R⁷, R⁸, R⁹, R¹⁰, R¹¹, and R¹² and which may be substituted has a substituted or unsubstituted aryl group as a substituent, the number of carbon atoms in the aryl group is preferably 6 to 22, more preferably 6 to 18, and still more preferably 6 to 10. Specifically, examples of the aryl group include a phenyl group, a naphthyl group, an anthracenyl group, and a phenanthrenyl group. As the substituent on the aryl group, an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴ is preferable, and a hydroxyl group or a carboxyl group is more preferable. Specifically, examples of the substituted aryl group include a hydroxyphenyl group, and a carboxyphenyl group.

In a case where the alkyl group having 1 to 18 carbon atoms which is represented by R⁴, R⁵, R⁶, R⁷, R⁸, R⁹, R¹⁰, R¹¹, and R¹² and which may be substituted has a substituted or unsubstituted heteroaryl group as a substituent, the number of carbon atoms in the heteroaryl group is preferably 1 to 12, and more preferably 1 to 6. Specifically, examples of the heteroaryl group include a pyridyl group, a pyrazolyl group, an imidazolyl group, a benzimidazolyl group, a thiazolyl group, and an oxazolyl group. As the substituent on the heteroaryl group, an alkyl group having 1 to 18 carbon atoms, a hydroxyl group, a carboxyl group, an amino group represented by -NR⁴⁵R⁴⁶, or a group represented by -O(CO)O-R⁴¹, - O(CO)-R⁴², -(CO)O-R⁴³, or -O-R⁴⁴ is preferable, and a hydroxyl group or a carboxyl group is more preferable. Specifically, examples of the substituted or unsubstituted heteroaryl group include a hydroxypyridyl group, a carboxypyridyl group, and a pyridonyl group.

As hydrocarbon group having 1 to 18 carbon atoms that is represented by R⁴¹, R⁴², R⁴³, R⁴⁴, R⁴⁵, and R⁴⁶, an alkyl group having 1 to 18 carbon atoms, an alkenyl group having 2 to 18 carbon atoms, or an alkynyl group having 2 to 18 carbon atoms is preferable, and an alkyl group having 1 to 18 carbon atoms or an alkenyl group having 2 to 18 carbon atoms is more preferable. The alkyl group having 1 to 18 carbon atoms may be linear or branched or may be chain-like or cyclic. The number of carbon atoms in the alkyl group having 1 to 18 carbon atoms is preferably 3 to 18, and more preferably 5 to 18. Specifically, examples thereof include a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a heptadecyl group, and an octadecyl group. The alkenyl group having 2 to 18 carbon atoms may be linear or branched or may be chain-like or cyclic. The number of carbon atoms in the alkenyl group having 2 to 18 carbon atoms is preferably 3 to 18, and more preferably 5 to 18. Specifically, examples thereof include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 2 to 18 carbon atoms may be linear or branched or may be chain-like or cyclic. The number of carbon atoms in the alkyl group having 1 to 18 carbon atoms is preferably 3 to 18, and more preferably 5 to 18. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group.

In a case where X represents -NR¹-, it is preferable that R¹ is a hydrocarbon group having 6 to 24 carbon atoms or a group represented by R²¹-L¹-R^{22_}. In this case, it is preferable that one of R² or R³ is a hydrogen atom and the other of R² or R³ is a hydrocarbon group having 6 to 24 carbon atoms or a group represented by R³¹-L²-R³²-.

In a case where X represents -O-, it is preferable that R² and R³ are each independently a hydrocarbon group having 6 to 24 carbon atoms or a group represented by R³¹-L²-R³²-.

It is preferable that R⁴, R⁶, R⁹, R¹⁰, R¹¹, and R¹² are a hydrogen atom.

It is preferable that R⁵ is a hydrogen atom, an alkyl group having 1 to 18 carbon atoms, an alkyl group having 1 to 18 carbon atoms which may be substituted with -O(CO)-R⁴² or -

(CO)O-R⁴³, an alkyl group having 1 to 18 carbon atoms which may be substituted with an aryl group, or an alkyl group having 1 to 18 carbon atoms which may be substituted with a hydroxyl group. In a case where R⁵ is an alkyl group, R⁵ may be linked to R⁴, R⁶, R¹⁰, and R¹² to form a ring which may contain an O atom. Particularly, it is preferable that R⁵ is an alkyl group having 1 to 18 carbon atoms, an alkyl group having 1 to 18 carbon atoms which may be substituted with -O(CO)-R⁴² or -(CO)O-R⁴³, an alkyl group having 1 to 12 carbon atoms which may be substituted with an aryl group, or an alkyl group having 1 to 8 carbon atoms which may be substituted with a hydroxyl group, and more preferable that R⁵ is an alkyl group having 1 to 18 carbon atoms or an alkyl group having 1 to 18 carbon atoms which may be substituted with -O(CO)-R⁴² or -(CO)O-R⁴³.

It is preferable that R⁷ and R⁸ are each independently a hydrogen atom, a hydrocarbon group having 1 to 18 carbon atoms, an alkyl group having 1 to 18 carbon atoms which may be substituted with -O(CO)-R⁴² or -(CO)O-R⁴³, an alkyl group having 1 to 8 carbon atoms which may be substituted with an aryl group, or an alkyl group having 1 to 8 carbon atoms which may be substituted with a hydroxyl group, or alternatively, R⁷ and R⁸ are linked to each other to form a 4- to 7-membered ring which may contain an O atom.

R⁵ is not linked to R⁷ or R⁸ and does not form a ring with R⁷ or R⁸.

a + b is preferably 1 or 2, and more preferably 1. c + d is preferably 1 or 2, and more preferably 1.

In a preferred embodiment, the lipid represented by Formula (2) is a lipid represented by Formula (3).

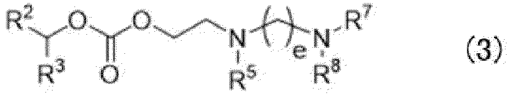

In the formula,
R² and R³ are each independently a hydrocarbon group having 3 to 24 carbon atoms which contains one or more unsaturated bonds,
R² and R³ are each independently a group represented by R³¹-L²-R³²-, or
one of R² or R³ is a group represented by R³¹-L²-R³²-, and the other is a hydrocarbon group having 3 to 24 carbon atoms,
where R³¹ represents a hydrocarbon group having 1 to 24 carbon atoms,
L² represents -O(CO)O-, -O(CO)-, -(CO)O-, -O-, or 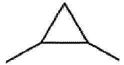 , and
R³² represents a divalent hydrocarbon linking group having 1 to 18 carbon atoms,
R⁵ represents an alkyl group having 1 to 18 carbon atoms which may be substituted with -O(CO)-R⁴² or -(CO)O-R⁴³, where R⁴² and R⁴³ each independently represent a hydrocarbon group having 1 to 18 carbon atoms,
R⁷ and R⁸ each independently represent an alkyl group having 1 to 4 carbon atoms, and
e represents 2 or 3.

In Formula (3), it is preferable that one of R² or R³ is a group represented by R³¹-L^{2_} R³²- and the other is a hydrocarbon group having 3 to 24 carbon atoms. In Formula (3), it is preferable that L² represents -O(CO)- or -(CO)O-.

The lipid represented by Formula (2) may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Among the above salts, preferred examples of the salt include pharmacologically acceptable salts.

Preferred specific examples of the lipid represented by Formula (2) can include 2-pentylheptyl 6-(2-(decanoyloxy)ethyl)-3-ethyl-12-hexyl-10-oxo-9,11-dioxa-3,6-diazahexadecan-16-oate (see WO2021/095876A).

The lipid represented by Formula (2) and a method for manufacturing the lipid are described in WO2019/23563 5A and WO2021/095876A.

In the lipid composition according to the embodiment of the present invention, the content of the lipid represented by Formula (2) or a salt thereof is preferably 20 mol% to 80 mol%, more preferably 30 mol% to 70 mol%, and still more preferably 40 mol% to 60 mol%, with respect to the total amount of the lipid.

### <Neutral lipid>

The lipid composition according to the embodiment of the present invention contains a neutral lipid.

The neutral lipid is preferably a zwitterionic lipid.

As the zwitterionic lipid, a phospholipid is preferable, and specific examples thereof include phosphatidylcholine, phosphatidylethanolamine, and sphingomyelin. As the phospholipid, a phospholipid having a choline group such as phosphatidylcholine is preferable. The zwitterionic lipid may be used either singly or in combination of a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), dioleoylphosphatidylcholine (DOPC), dilauroylphosphatidylcholine (DLPC), and 1-palmitoyl-2-oleoylphosphatidylcholine (POPC). Among them, dimyristoylphosphatidylcholine (DMPC), distearoylphosphatidylcholine (DSPC), and dilauroylphosphatidylcholine (DLPC) are preferable, and distearoylphosphatidylcholine (DSPC) is particularly preferable.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine and diphytanylphosphatidylethanolamine.

The sphingomyelin (SM) is not particularly limited, and examples thereof include egg yolk-derived sphingomyelin, and milk-derived sphingomyelin.

In the lipid composition according to the embodiment of the present invention, the content of the neutral lipid is preferably 1 to 30 mol%, more preferably 5 to 25 mol%, and still more preferably 7 to 23 mol% with respect to the total lipids.

### <Lipid having nonionic hydrophilic polymer>

The lipid composition according to the embodiment of the present invention contains a lipid having a nonionic hydrophilic polymer.

The lipid having a nonionic hydrophilic polymer preferably contains an acyl group, and the carbon chain length of the acyl group is preferably 8 to 26.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable.

The lipid having a nonionic hydrophilic polymer is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, monoacylglycerol PEG or diacylglycerol PEG is preferable.

The number of carbon atoms in the alkyl chain of the lipid having a nonionic hydrophilic polymer is preferably 8 to 26 and more preferably 10 to 22.

A weight-average molecular weight of the nonionic hydrophilic polymer is preferably 100 to 10,000, more preferably 500 to 5,000, and still more preferably 750 to 3,000.

The nonionic hydrophilic polymer chain may be branched or may have a substituent such as a hydroxymethyl group.

Preferred examples of the lipid having a nonionic hydrophilic polymer include DMG-PEG2000 (also referred to as DMG-PEG) having the following structure, PEG stearic acid, and PEG stearyl ether.

Particularly preferred specific examples of the lipid having a nonionic hydrophilic polymer are shown below.

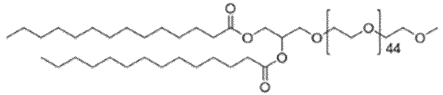

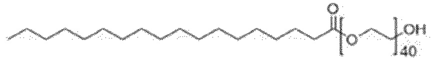

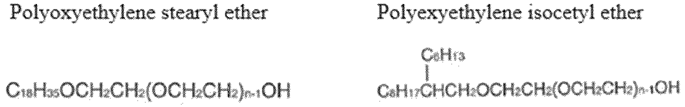

In the lipid composition according to the embodiment of the present invention, the blending amount of the lipid having a nonionic hydrophilic polymer is preferably 0.1 to 10 mol%, more preferably 0.3 to 8 mol%, still more preferably 0.5 to 5 mol%, and particularly preferably 1 to 3 mol% with respect to the total lipids.

### <Nucleic acid>

The lipid composition according to the embodiment of the present invention contains a nucleic acid. Examples of the nucleic acid include a plasmid, a single-stranded DNA, a double-stranded DNA, a small interfering RNA (siRNA), a micro RNA (miRNA), an mRNA, an antisense oligonucleotide (also referred to as an ASO), a ribozyme, an aptamer, a decoy nucleic acid, a gRNA used in genome editing, and the like, any of which may be contained. In addition, the lipid composition may contain a modified nucleic acid.

In the lipid composition according to the embodiment of the present invention, the weight ratio of the lipids to the nucleic acid is preferably 5 to 100, more preferably 5 to 70, still more preferably 5 to 40, and particularly preferably 5 to 35.

### <Method for manufacturing lipid composition>

The method for manufacturing the lipid composition according to the embodiment of the present invention will be described.

The method for manufacturing the lipid composition is not limited, and for example, the lipid composition can be manufactured by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to dryness using an evaporator under reduced pressure or subjected to spray drying using a spray drier, so that a dried mixture containing a lipid is prepared, and then the mixture is added to an aqueous solvent and further emulsified using the above-described emulsifying machine or the like.

One example of the method for manufacturing the lipid composition is a method including
Step (a) of dissolving the lipid components in an organic solvent to obtain an oil phase,
Step (b) of mixing the oil phase obtained in Step (a) with a water phase containing a nucleic acid,
Step (c) of diluting the mixed solution containing the oil phase and the water phase obtained in Step (b) to obtain a dispersion liquid of lipid composition containing the nucleic acid, and
Step (d) of removing the organic solvent from the dispersion liquid of lipid composition obtained in Step (c).

Step (a) includes a process of dissolving the lipid components in an organic solvent (an alcohol such as ethanol, an ester, or the like). The total lipid concentration is not particularly limited, but is generally 1 mmol/L to 100 mmol/L, preferably 5 mmol/L to 50 mmol/L, and more preferably 10 mmol/L to 30 mmol/L.

In Step (b), the water phase can be obtained by dissolving a nucleic acid (for example, siRNA, an antisense oligonucleotide, or mRNA) in water or a buffer solution. If necessary, a component such as an antioxidant can be added. The mixing ratio (volume ratio) of water phase:oil phase is preferably 5:1 to 1:1 and more preferably 4:1 to 2:1.

In Step (c), the mixed solution can be diluted with water or a buffer solution (for example, phosphate buffered saline (PBS)).

In Step (d), the method of removing the organic solvent from the dispersion liquid of lipid composition is not particularly limited, and a general method can be used. For example, the organic solvent can be removed by dialyzing the dispersion liquid with the phosphate buffered saline.

The lipid composition can be subjected to sizing if necessary. The method of sizing is not particularly limited, and the particle size can be reduced by using an extruder or the like.

### <Regarding lipid composition>

The lipid composition according to the embodiment of the present invention may be a lipid particle. The lipid particle means a particle composed of a lipid and includes a composition having any of structures selected from a lipid aggregate in which lipids are aggregated (for example, a lipid nanoparticle or the like), a micelle, or a liposome, but the structure of the lipid particle is not limited to these structures as long as the lipid particle is a composition containing a lipid.

The form of the lipid composition can be checked by electron microscopy, structural analysis using X-rays, or the like. For example, by a method using Cryo transmission electron microscopy (CryoTEM method), it is possible to check whether or not a lipid particle is, such as a liposome, a bimolecular lipid membrane structure (lamella structure) and a structure having an inner water layer, whether or not a lipid particle has a structure having a core with a high electron density inside the particle and packed with constituent components including a lipid, and the like. The X-ray small angle scattering (SAXS) measurement also makes it possible to check whether or not a lipid particle has a bimolecular lipid membrane structure (lamella structure).

In the case where the lipid composition according to the embodiment of the present invention is particles, the particle size of the particles is not particularly limited, but is preferably 10 to 1,000 nm, more preferably 30 to 500 nm, and still more preferably 50 to 250 nm. The particle size of the lipid particles can be measured by a general method (for example, a dynamic light scattering method, a laser diffraction method, or the like).

### <Use of lipid composition>

As one example for utilizing the lipid composition according to the embodiment of the present invention, a nucleic acid (for example, a gene) can be introduced into a cell by introducing the lipid composition containing the nucleic acid into the cell. That is, the lipid composition according to the embodiment of the present invention can be used as a composition for introducing a nucleic acid into a cell.

In addition, the lipid composition according to the embodiment of the present invention can be used as a pharmaceutical composition for nucleic acid delivery in vivo.

In addition, in a case where the lipid composition according to the embodiment of the present invention contains a nucleic acid for a pharmaceutical use, the lipid composition can be administered to a living body as a nucleic acid drug. In a case where the lipid composition in the present invention is used as a nucleic acid drug, the lipid composition according to the embodiment of the present invention can be administered to a living body singly or by being mixed with a pharmaceutically acceptable carrier (for example, a dosing medium such as physiological saline or a phosphate buffer solution). That is, the lipid composition according to the embodiment of the present invention may further contain a pharmaceutically acceptable carrier.

The concentration of the lipid composition in the mixture with a pharmaceutically acceptable carrier is not particularly limited, and can be set to 0.05% by mass to 90% by mass in general. In addition, other pharmaceutically acceptable additives, for example, a pH adjusting buffer and an osmotic pressure adjusting agent, may be added to the nucleic acid drug containing the lipid composition according to the embodiment of the present invention.

The route of administration in a case of administering the lipid composition according to the embodiment of the present invention is not particularly limited, and the lipid composition can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the administration method, intravenous injection, subcutaneous injection, intracutaneous injection, or intramuscular injection is preferable. As for the administration, it is preferable to deliver the nucleic acid by topical administration in vivo. The lipid composition according to the embodiment of the present invention can also be administered by being directly injected into the affected area.

The dosage form of the lipid composition according to the embodiment of the present invention is not particularly limited. In a case of performing oral administration, the lipid composition according to the embodiment of the present invention can be used in the form of tablets, troches, capsules, pills, suspension, syrup, or the like by being combined with an appropriate excipient. In addition, pharmaceutical preparation suitable for parenteral administration can appropriately contain an antioxidant, a buffer, a bacteriostat, and additives such as an isotonic sterile injection, a suspending agent, a solubilizer, a thickener, a stabilizer, or a preservative.

### <Nucleic acid delivery carrier>

The lipid composition according to the embodiment of the present invention can retain a nucleic acid at a high encapsulation rate and thereby is extremely useful as a nucleic acid delivery carrier. According to the nucleic acid delivery carrier using the present invention, the nucleic acid and the like can be introduced into the cells, for example, by mixing the obtained lipid composition with the nucleic acid and the like and transfecting the mixture in vitro or in vivo. In addition, the nucleic acid delivery carrier using the present invention is also useful as a nucleic acid delivery carrier in nucleic acid drugs. That is, the lipid composition according to the embodiment of the present invention is useful as a composition for nucleic acid delivery in vitro or in vivo (preferably in vivo).

Next, the present invention will be described based on examples, but the present invention is not limited thereto.

### Examples

Unless otherwise specified, for the purification by column chromatography, an automatic purification device ISOLERA (Biotage), a medium pressure separation and purification device Purif-espoir-2 (Shoko Science Co., Ltd.), or a medium pressure liquid chromatograph YFLC W-prep 2XY (Yamazen Corporation) was used.

Unless otherwise specified, as a carrier for silica gel column chromatography, Chromatorex Q-Pack SI 50 (FUJI SILYSIA CHEMICAL LTD.) or HIGH FLASH COLUMN W001, W002, W003, W004, or W005 (Yamazen Corporation) was used.

NMR spectra were measured using a Bruker AVNE0400 (manufactured by Bruker Corporation) and using tetramethylsilane as an internal standard, and all δ values were shown in ppm.
clogP was calculated using ChemDraw Professional Version: 19.1.0.8 (manufactured by PerkinElmer, Inc.).

### [Synthesis Example 1]

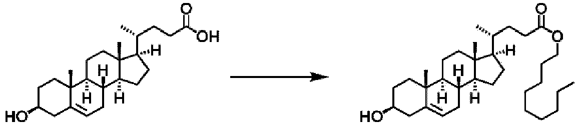

Potassium carbonate (1.66 g) was added to a mixture of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoic acid (3.00 g), 1-bromononane (1.99 g), and N,N-dimethylformamide (21 mL), and the mixture was stirred at 60°C for 4 hours. Ethyl acetate (42 mL) and water (42 mL) were added to the reaction mixture, the organic layer was separated, washed twice with water (42 mL), and then dried over anhydrous sodium sulfate (9g), and the solvent was distilled off under reduced pressure. Ethanol (60 mL) was added to the obtained residue, the mixture was stirred at 60°C for 5 minutes, and then cooled to room temperature, water (30 mL) was added thereto, and the mixture was stirred at 0°C for 30 minutes. Solids were collected by filtration, washed with a mixed solution of ethanol (30 mL) and water (15 mL), and then dried under reduced pressure, thereby obtaining nonyl(R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoate (compound 1, 3.80 g) as a white solid.

¹H-NMR(CDCl₃) δ: 5.37-5.33 (1H, m), 4.05 (2H, t, J = 6.8 Hz), 3.58-3.46 (1H, m), 2.40-2.15 (4H, m), 2.05-1.92 (2H, m), 1.92-1.73 (4H, m), 1.66-0.82 (38H), 0.68 (3H, s).
clogP: 10.725

### [Synthesis Example 2]

Compounds 2, 4, 5, and 8 to 14 were obtained by the same method as in Synthesis Example 1.

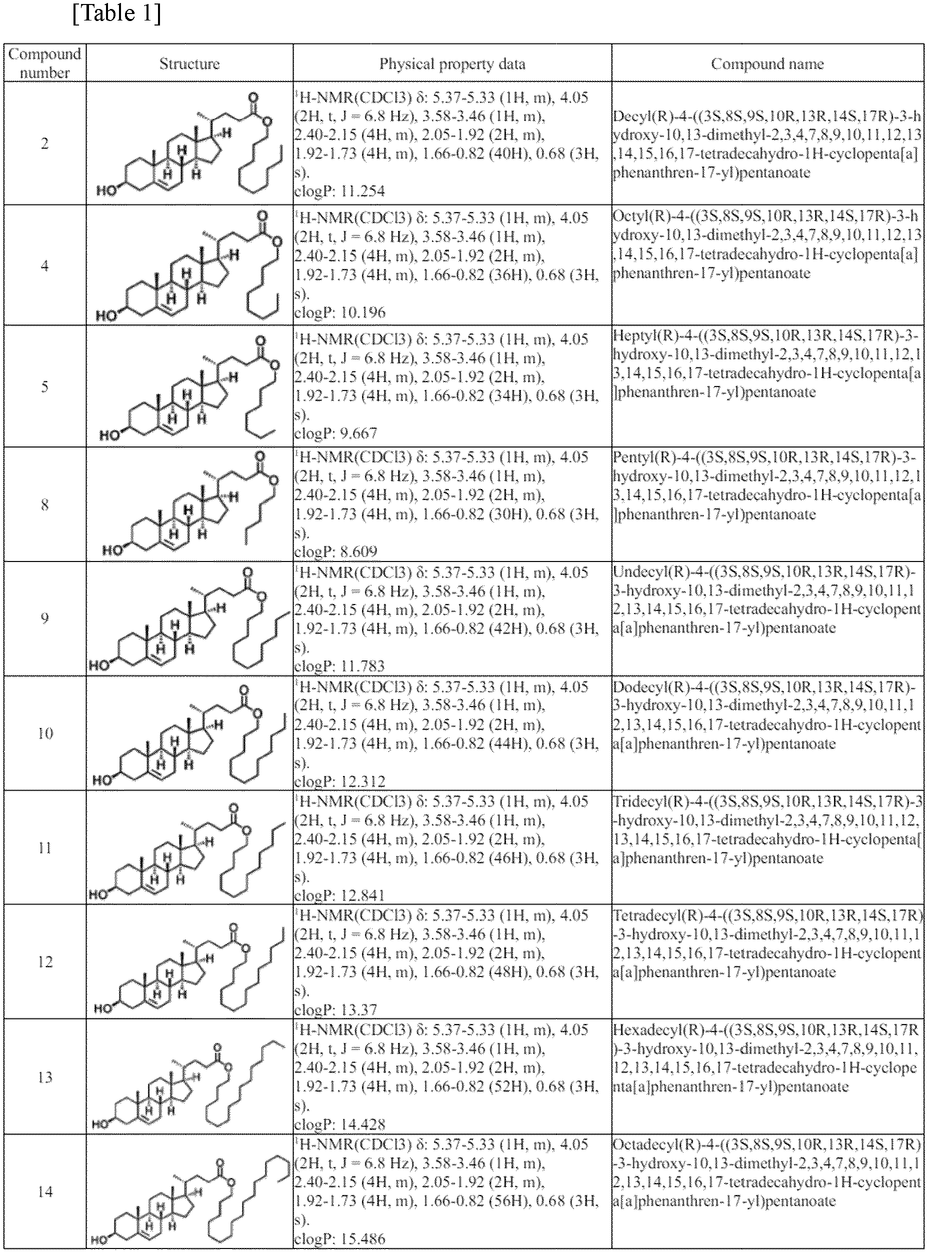

### [Synthesis Example 3]

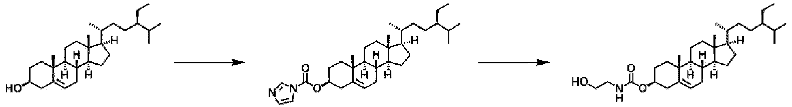

1,1'-Carbonyldiimidazole (0.88 g) was added to a tetrahydrofuran (7.5 mL) solution of β-sitosterol (1.50 g), and the mixture was stirred at 30°C for 4 hours. Water (7.5 mL), hexane (7.5 mL), and ethyl acetate (7.5 mL) were added to the reaction mixture, the organic layer was separated, and then washed with water (7.5 mL) and a saturated aqueous sodium chloride solution (3 mL), and dried over anhydrous sodium sulfate, and the solvent was distilled off under reduced pressure, thereby obtaining (3S,8S,9S,10R,13R,14S,17R)-17-((2R,5R)-5-ethyl-6-methylheptan-2-yl)-10,13-dimethyl-2,3,4,7,8,9,10,11,12,13,14,15,16,17-tetradecahydro-1H-cyclopenta[a]phenanthrene-3-yl 1H-imidazole-1-carboxylate (1.84 g) as a white solid.

¹H-NMR(CDCl₃) δ: 8.14-8.12 (1H, m), 7.43-7.41 (1H, m), 7.07-7.05 (1H, m), 5.47-5.42 (1H, m), 4.89-4.78 (1H, m), 2.50 (2H, d, J = 7.76 Hz), 2.08-0.76 (42H, m), 0.69 (3H, s).

2-Aminoethanol (0.66 g) and N,N-diisopropylethylamine (1.40 g) were added to a mixture of (3S,8S,9S,10R,13R,14S,17R)-17-((2R,5R)-5-ethyl-6-methylheptan-2-yl)-10,13-dimethyl-2,3,4,7,8,9,10,11,12,13,14,15,16,17-tetradecahydro-1H-cyclopenta[a]phenanthren-3-yl 1H-imidazole-1-carboxylate (1.84 g), tetrahydrofuran (9.2 mL), and acetonitrile (4.6 mL), and the mixture was stirred at 30°C for 4 hours. Water (15 mL), ethyl acetate (10 mL), and hexane (10 mL) were added to the reaction mixture, and the organic layer was separated and then washed with water (10 mL) and a saturated aqueous sodium chloride solution. The organic layer was dried over anhydrous sodium sulfate, the solvent was distilled off under reduced pressure, hexane (10 mL) was added to the obtained residue, and the mixture was stirred at 40°C for 5 minutes. The mixture was cooled to room temperature, and the solid was collected by filtration, washed with hexane, and dried under reduced pressure, thereby obtaining (3S,8S,9S,10R,13R,14S,17R)-17-((2R,5R)-5-ethyl-6-methylheptan-2-yl)-10,13-dimethyl-2,3,4,7,8,9,10,11,12,13,14,15,16,17-tetradecahydro-1H-cyclopenta[a]phenanthrene-3-yl(2-hydroxyethyl)carbamate (compound 3, 1.73 g) as a white solid.

¹H-NMR(CDCl₃) δ: 5.40-5.35 (1H, m), 5.00 (1H, brs), 4.56-4.45 (1H, m), 3.73 (2H, brs), 3.38-3.28 (2H, m), 2.40-2.15 (3H, m), 2.05-1.78 (5H, m), 1.73-0.76 (36H, m), 0.68 (3H, s).
clogP: 10.4878

### [Synthesis Example 4]

Compounds 6, 7, and 17 were obtained by the same method as in Synthesis Example 3.

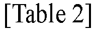

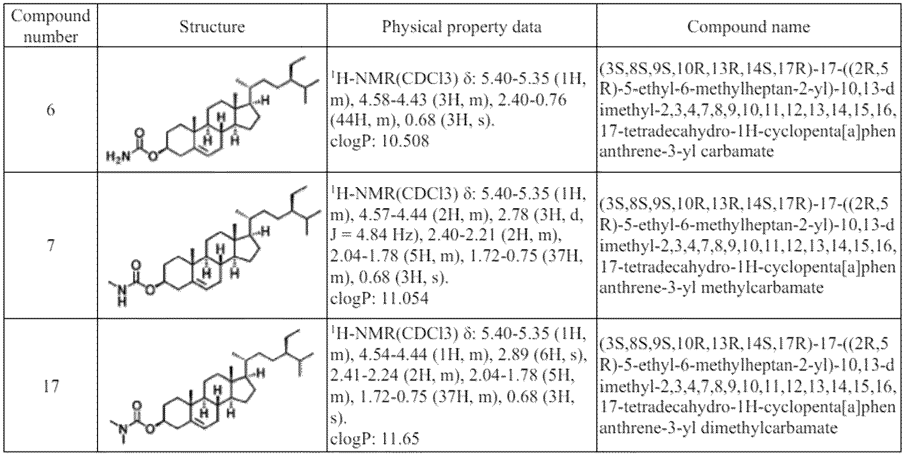

### [Synthesis Example 5]

### Synthesis of Compound 15

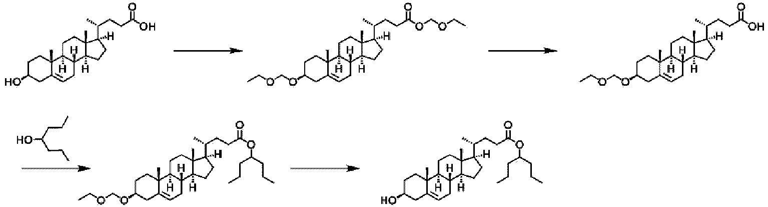

Chloromethyl ethyl ether (4.5 mL) was added dropwise to a mixture of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoic acid (6.00 g), N,N-diisopropylethylamine (13.5 mL), and dichloromethane (60 mL) under ice cooling, and the mixture was stirred at the same temperature for 1 hour. Water (60 mL) was added dropwise to the reaction mixture under ice cooling, and then the organic layer was separated and the water layer was extracted with a mixed solution of hexane (15 mL) and ethyl acetate (15 mL). The organic layer and the extraction solution were combined, the solvent was distilled off under reduced pressure, and the obtained residue was purified by silica gel chromatography (ethyl acetate-hexane), thereby obtaining ethoxymethyl(R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoate (6.74 g) as a colorless oily substance.

¹H-NMR(CDCl₃) δ: 5.37-5.32 (1H, m), 5.28 (2H, s), 4.74 (2H, s), 3.68 (2H, q, J = 7.08 Hz), 3.61 (2H, q, J = 7.08 Hz), 3.49-3.39 (1H, m), 2.44-2.20 (4H, m), 2.04-0.82 (33H, m), 0.68 (3H, s).

An aqueous (10 mL) solution of potassium hydroxide (2.72 g) was added to a mixture of ethoxymethyl (R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoate (6.74 g), tetrahydrofuran (20 mL), and ethanol (20 mL), and the mixture was stirred at 40°C for 2 hours. A 5 mol/L aqueous hydrochloric acid solution (6 mL) was added dropwise to the reaction mixture under ice cooling, ethyl acetate (40 mL) was added thereto, the organic layer was separated, and then the organic layer was washed with water and a saturated aqueous sodium chloride solution. After drying over anhydrous sodium sulfate, the solvent was distilled off under reduced pressure, thereby obtaining (R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoic acid (5.29 g) as a white solid.

¹H-NMR(CDCl₃) δ: 5.37-5.32 (1H, m), 4.74 (2H, s), 3.61 (2H, q, J = 7.08 Hz), 3.49-3.39 (1H, m), 2.44-2.20 (4H, m), 2.04-0.82 (30H, m), 0.68 (3H, s).

1-Ethyl-3-(3-dimethylaminopropyl)carbodiimide hydrochloride (0.33 g) and 4-dimethylaminopyridine (0.14 g) were added to a mixture of (R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoic acid (0.50 g), 4-heptanol (0.15 g), N,N-diisopropylethylamine (1.0 mL), and dichloromethane (5.0 mL), and the mixture was stirred at 40°C for 1 hour. Water (5.0 mL) was added to the reaction mixture, the organic layer was separated, and the water layer was extracted with a mixed solution of hexane and ethyl acetate. The organic layer and the extraction solution were combined, the solvent was distilled off under reduced pressure, and the obtained residue was purified by silica gel chromatography (ethyl acetate-hexane), thereby obtaining heptan-4-yl(R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoate (0.61 g) as a colorless oily substance.

¹H-NMR(CDCl₃) δ: 5.37-5.32 (1H, m), 4.74 (2H, s), 4.94-4.86 (2H, m), 3.61 (2H, q, J = 7.08 Hz), 3.49-3.39 (1H, m), 2.38-2.15 (4H, m), 2.04-0.82 (43H, m), 0.68 (3H, s).

Trifluoroacetic acid (1.0 mL) was added to a mixture of heptan-4-yl(R)-4-((3S,8S,9S,10R,13R,14S,17R)-3-(ethoxymethoxy)-10,13-dimethyl-2,3,4,7,8,9,10,11,12,13,14,15,16,17-tetradecahydro-1H-cyclopenta[a]phenanthren-17-yl)pentanoate (0.61 g), water (0.1 mL), and dichloromethane (4.0 mL) under ice cooling, and the mixture was stirred at room temperature for 1 hour. Sodium hydrogen carbonate (3.5 g), water (8 mL), ethyl acetate (4 mL), and hexane (4 mL) were added to the reaction mixture under ice cooling, the organic layer was separated and dried over anhydrous sodium sulfate, and the solvent was distilled off under reduced pressure. The obtained residue was purified by silica gel chromatography (ethyl acetate-hexane), and heptan-4-yl(R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoate (compound 15, 0.34 g) as a colorless oily substance was obtained.

¹H-NMR(CDCl₃) δ: 5.37-5.33 (1H, m), 4.94-4.85 (1H, m), 3.58-3.46 (1H), 2.40-2.15 (4H, m), 2.05-1.92 (2H, m), 1.92-1.73 (4H, m), 1.66-0.82 (35H), 0.68 (3H, s).
clogP: 9.447

### [Synthesis Example 6]

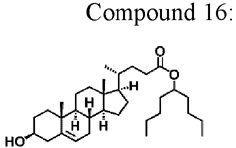

Nonan-5-yl(R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoate (compound 16) as a colorless oily substance was obtained by the same method as in Synthesis Example 5, except that in Synthesis Example 5, 5-nonanol was used instead of 4-heptanol.

¹H-NMR(CDCl₃) δ: 5.37-5.33 (1H, m), 4.94-4.85 (1H, m), 3.58-3.46 (1H), 2.40-2.15 (4H, m), 2.05-1.92 (2H, m), 1.92-1.73 (4H, m), 1.66-0.82 (39H), 0.68 (3H, s).
clogP: 10.505

### (PTEN antisense oligonucleotide information)

Phosphatase and Tensin Homolog Deleted from Chromosome 10 (PTEN) is an enzyme that catalyzes a dephosphorylation reaction of phosphatidylinositol 3,4,5-triphosphate, which is an inositol phospholipid.

An antisense oligonucleotide nucleic acid (PTEN ASO) for a PTEN protein was purchased from Ajinomoto Bio-Pharma. and Hokkaido System Science Co., Ltd. PTEN ASO is an oligonucleotide consisting of 20 bases which are connected by a phosphodiester bond, and the sequence of which is described below.

5’-
C(m)^T(m)^G(m)^C(m)^T(m)^a^g^c^c^t^c^t^g^g^a^T(m)^T(m)^T(m)^G(m)^A(m)-3’

Provided that a represents 2'-deoxyadenosine, g represents 2'-deoxyguanosine, t represents thymidine, and c represents 2'-deoxy-5-methylcytidine. (m) represents 2'-O-methoxyethyl (2'-MOE) modification, A(m) represents 2'-MOE-adenosine, G(m) represents 2'-MOE-guanosine, T(m) represents 2'-MOE-thymidine, and C(m) represents 2'-MOE-5-methylcytidine. ^ represents phosphorothioate.

### (Preparation of PTEN ASO-LNP)

The first lipid (ionizable lipid), sterol, phospholipid, and polyethylene glycol lipid (PEG lipid), which are shown in Table 3 were dissolved in ethanol at the molar ratio shown in Table 3 such that the total lipid concentration was 20 mmol/L to obtain the oil phase.

The components used are shown below.
1,2-Distearoyl-sn-glycero-3-phosphocholine (DSPC, product name: COATSOME MC-8080, NOF corporation)
Cholesterol (Product name: Cholesterol HP, Nippon Seika Co., Ltd.)
1,2-Dimyristoyl-rac-glycero-3-methoxypolyethylene glycol (DMG-PEG2000, product name: SUNBRIGHT (register trademark) GM-020, NOF corporation)

The structure of DMG-PEG2000 (also referred to DMG-PEG) is shown below.

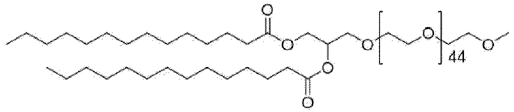

DMPE-PEG2000 (N-(Methylpolyoxyethylene oxycarbonyl)-1,2-dimyristoyl-sn-glycero-3-phosphoethanolamine, sodium salt) (product name: SUNBRIGHT (register trademark) PM-020CN, NOF corporation)

The structure of DMPE-PEG2000 is shown below.

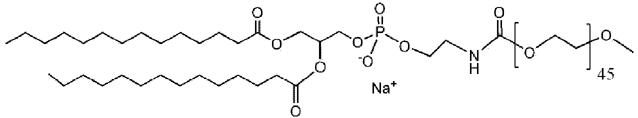

The compound A is 2-pentylheptyl 6-(2-(decanoyloxy)ethyl)-3-ethyl-12-hexyl-10-oxo-9,11-dioxa-3,6-diazahexadecane-16-oate (see Example 135 of WO2021/095876A).

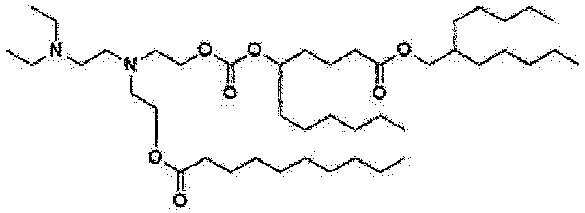

The compound B is 2-butyloctyl 3-ethyl-12-hexyl-6-(2-(octanoyloxy)ethyl)-10-oxo-9,11-dioxa-3,6-diazahenicosane-21-oate (see Example 88 of WO2019/235635A).

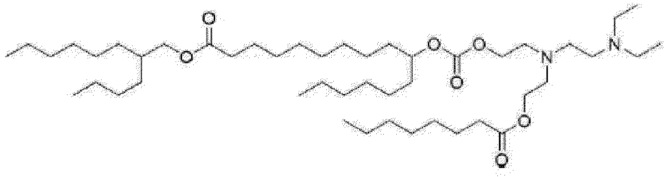

5 mg of PTEN ASO was dissolved in 1 mL of sterile water and diluted with a 10 mmol/L acetate buffer having a pH of 4 such that the nucleic acid concentration was 54.6 µmol/L, to obtain a water phase. Then the water phase and the oil phase were mixed together with a micromixer (see JP5288254B) using a syringe pump such that the volume ratio of water phase:oil phase was 3: 1, and the mixed solution was two-fold diluted with a phosphate buffered saline (PBS), thereby obtaining a nucleic acid lipid particle dispersion.

Table 3 shows the molar ratio of the first lipid (the lipid represented by Formula (2) or a salt of the lipid), the phospholipid (the neutral lipid), the sterol (the compound represented by Formula (1)), and the PEG lipid (the lipid having a nonionic hydrophilic polymer) in the lipid composition. Table 3 also shows the mass ratio of the nucleic acid to the total lipids at the time of mixing.

**[Table 3]**

| | First lipid | Sterol | Phospholipid | PEG lipid | Composition ratio of lipid (mol%) | | | | Mass ratio of nucleic acid to total lipids |
|---|---|---|---|---|---|---|---|---|---|
| | | | | | First lipid | Phospholipid | Sterol | PEG lipid. | |
| | | | | | | | Cholesterol | | |
| Example 1 | Compound A | Compound 1 | DSPC | DMG-PEG2000 | 50 | 10 | 38.5 | 1.5 | 12.7 |
| Example 2 | Compound A | Compound 2 | DSPC | DMG-PEG2000 | 50 | 10 | 38.5 | 1.5 | 12.7 |
| Example 3 | Compound A | Compound 3 | DSPC | DMG-PEG2000 | 50 | 10 | 38.5 | 1.5 | 12.7 |
| Example 4 | Compound A | Compound 4 | DSPC | DMG-PEG2000 | 50 | 10 | 38.5 | 1.5 | 12.7 |
| Example 5 | Compound A | Compound 5 | DSPC | DMG-PEG2000 | 50 | 10 | 38.5 | 1.5 | 12.7 |
| Example 6 | Compound A | Compound 6 | DSPC | DMG-PEG2000 | 50 | 10 | 38.5 | 1.5 | 12.7 |
| Example 7 | Compound A | Compound 7 | DSPC | DMG-PEG2000 | 50 | 10 | 38.5 | 1.5 | 12.7 |
| Comparative example 1 | Compound A | Cholesterol | DSPC | DMG-PEG2000 | 50 | 10 | 38.5 | 1.5 | 12.7 |
| Comparative example 2 | Compound A | Compound 8 | DSPC | DMG-PEG2000 | 50 | 10 | 38.5 | 1.5 | 12.7 |
| Example 8 | Compound A | Compound 9 | DSPC | DMG-PEG2000 | 50 | 10 | 38.5 | 1.5 | 12.7 |
| Example 9 | Compound A | Compound 10 | DSPC | DMG-PEG2000 | 50 | 10 | 38.5 | 1.5 | 12.7 |
| Example 10 | Compound A | Compound 11 | DSPC | DMG-PEG2000 | 50 | 10 | 38.5 | 1.5 | 12.7 |
| Example 11 | Compound A | Compound 12 | DSPC | DMG-PEG2000 | 50 | 10 | 38.5 | 1.5 | 12.7 |
| Example 12 | Compound A | Compound 13 | DSPC | DMPE-PEG2000 | 50 | 10 | 38.5 | 1.5 | 12.7 |
| Example 13 | Compound A | Compound 14 | DSPC | DMG-PEG2000 | 50 | 10 | 38.5 | 1.5 | 12.7 |
| Example 14 | Compound A | Compound 15 | DSPC | DMG-PEG2000 | 50 | 10 | 38.5 | 1.5 | 12.7 |
| Example 15 | Compound A | Compound 16 | DSPC | DMG-PEG2000 | 50 | 10 | 38.5 | 1.5 | 12.7 |
| Example 16 | Compound A | Compound 17 | DSPC | DMG-PEG2000 | 50 | 10 | 38.5 | 1.5 | 12.7 |

### <Measurement of particle size>

The particle size and polydispersion index of the lipid particles were measured by 5-fold diluting the dispersion liquid of lipid particle with phosphate buffered saline (PBS) using Zeta-potential and Particle size Analyzer ELS-Z2 (Otsuka Electronics Co., Ltd.). The measurement results are shown in Table 4.

### <Evaluation of encapsulation rate of PTEN ASO>

### (Quantification of total nucleic acid concentration)

30 µL of 3 mol/L aqueous sodium acetate solution and 9 µL of glycogen were added to 60 µL of the lipid particles retaining nucleic acids, and then 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop ND1000 (Thermo Fischer Scientific), thereby quantifying the total nucleic acid concentration.

### (Quantification of nucleic acid concentration in outer water phase)

The nucleic acid concentration was quantified using a Quant-iT RiboGreen RNA Assay Kit (Thermo Fischer Scientific) according to the protocol. First, a 20× TE buffer included in the above kit was diluted with water, thereby obtaining a 1× TE buffer. TE represents Tris/EDTA (ethylenediaminetetraacetic acid). In order to quantify only the nucleic acid in the outer water phase, the dispersion liquid of lipid particles retaining nucleic acids was 50-fold diluted with the 1× TE buffer.

100 µL of the 10,000-fold diluted dispersion liquid of lipid particles was put into a 96-well plate, then 100 µL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the nucleic acid concentration in the outer water phase.

### (Calculation of encapsulation rate)

By using the quantification results of the total nucleic acid concentration and the nucleic acid concentration in the outer water phase that were obtained through the above steps, the nucleic acid encapsulation rate of the nucleic acid lipid particles was calculated according to the following Equation. Nucleic acid encapsulation rate (%) = (total nucleic acid concentration - nucleic acid concentration in outer water phase)/total nucleic acid concentration × 100

The calculation results are shown in Table 4.

**[Table 4]**

| | Cumulant particle size (nm) | Polydispersion index | Encapsulation rate (%) |
|---|---|---|---|
| Example 1 | 63.6 | 0.069 | 91 |
| Example 2 | 66.6 | 0.074 | 91 |
| Example 3 | 103.0 | 0.091 | 95 |
| Example 4 | 68.1 | 0.010 | 89 |
| Example 5 | 67.6 | 0.026 | 92 |
| Example 6 | 110.0 | 0.208 | 85 |
| Example 7 | 60.0 | 0.033 | 81 |
| Comparative example 1 | 61.4 | 0.042 | 100 |
| Comparative example 2 | 62.9 | 0.073 | 89 |
| Example 8 | 66.1 | 0.052 | 89 |
| Example 9 | 61.2 | 0.041 | 88 |
| Example 10 | 68.9 | 0.198 | 84 |
| Example 11 | 91.1 | 0.275 | 79 |
| Example 12 | 131.0 | 0.150 | 89 |
| Example 13 | 119.0 | 0.131 | 82 |
| Example 14 | 58.1 | 0.071 | 75 |
| Example 15 | 59.1 | 0.084 | 72 |
| Example 16 | 55.6 | 0.109 | 72 |

### <Evaluation of PTEN mRNA knockdown in vitro>

### (Cells used for evaluation)

In an in vitro test using SH-SY5Y cells (American Type Culture Collection), E-MEM (gibco), Ham's F12 (gibco), FBS (gibco), Penicillin-streptomycin (gibco), and NEAA (FUJIFILM Wako Pure Chemical Corporation) were mixed at a ratio of 41.5:41.5:15:1:1 and the mixture was used as a culture medium.

### (Quantification of PTEN mRNA by PCR)

The measurement of PTEN protein mRNA was performed according to the protocol of TaqMan (register trademark) Fast Advanced Cells-to-CT^{™} Kit (Thermo Fischer Scientific). A dispersion liquid of nucleic acid lipid particles prepared such that the final concentration was 500 nmol/L as the ASO concentration, Naked ASO or PBS was added to the SH-SY5Y cells. After exposure for 24 hours under the control of 37°C and 5% CO₂, the culture supernatant was removed, and the mixture was washed once with PBS at 4°C. After removing the PBS, a lysis solution was added at 50 µL/well, and the mixture was stood still for 5 minutes at room temperature to obtain a cell lysate. For the cell lysate, TaqMan (register trademark) Fast Advanced Cells-to-CT^{™} Kit (Thermo Fischer scientific), and Hs02621230_s1, FAM/MGB (Thermo Fischer Scientific) and Human GAPDH Endogenous Control (VIC (register trademark)/MGB, Thermo Fischer Scientific) as a PCR reaction reagent were used to perform reverse transcription and PCR reaction.

The PTEN mRNA level of each sample was calculated by the ΔΔCt method. Specifically, the Ct value of GAPDH is subtracted from the Ct value of PTEN to calculate the ΔCt value of each sample. The average value of the ΔCt values of the PBS treatment group was subtracted from the calculated ΔCt value to calculate the ΔΔCt value. The PTEN mRNA expression ratio was calculated from each ΔΔCt value. The results are shown in Table 5.

**[Table 5]**

| PTEN mRNA expression ratio with respect to control in SH-SY5Y cell | |
|---|---|
| | SH-SY5Y cell |
| Example 1 | 0.09 |
| Example 2 | 0.24 |
| Example 3 | 0.04 |
| Example 4 | 0.15 |
| Example 5 | 0.44 |
| Comparative example 1 | 0.59 |
| Comparative example 2 | 0.67 |
| Example 8 | 0.39 |
| Example 9 | 0.51 |
| Example 10 | 0.18 |
| Example 11 | 0.56 |
| Example 13 | 0.50 |
| Example 16 | 0.50 |

### <Preparation of nucleic acid lipid particles>

The compound A or the compound B, 1,2-distearoyl-sn-glycero-3-phosphocholine (DSPC, product name: COATSOME (R) MC-8080; manufactured by NOF corporation), a compound described in Table 6, and 1,2-dimyristoyl-rac-glycero-3-methoxypolyethylene glycol-2000 (DMG-PEG2000, product name: SUNBRIGHT (R) GM-020; manufactured by NOF corporation) were dissolved in ethanol at a molar ratio of 50:10:38.5:1.5 such that the total lipid concentration was 12.5 mmol/L, thereby obtaining an oil phase.

FLuc mRNA (product name: CleanCap FLuc mRNA; manufactured by TriLink BioTechnologies.) was diluted with a 50 mmol/L citrate buffer having a pH 4 such that the weight ratio of total lipid concentration after mixing an oil phase and a water phase, to mRNA concentration is about 20:1, thereby obtaining the water phase. Then, the water phase and the oil phase were mixed together using NanoAssemblr (Precision NanoSystems) such that the volume ratio of water phase:oil phase was 3:1, and the mixed solution was 2-fold diluted with phosphate buffered saline (PBS), thereby obtaining a dispersion liquid of mRNAlipid particles. The dispersion liquid was dialyzed with 20 mM Tris buffer solution containing 8% sucrose and having a pH of 7.4 using a dialysis cassette (Slide-A-Lyzer G2, MWCO: 10 kD, Thermo Fisher Scientific) to remove ethanol, thereby obtaining FLuc mRNA-encapsulating lipid particles.

### <Measurement of particle size>

The particle size of the mRNA-encapsulating lipid particle was measured using a Zeta Potential and Particle Size Analyzer NanoSAQLA (Otsuka Electronics Co., Ltd.) after diluting the lipid particles 5 times with phosphate buffered saline (PBS). The results are shown in Table 6.

### <Evaluation of encapsulation rate of mRNA>

### (Quantification of total mRNA concentration)

FLuc mRNA was diluted with MilliQ water to prepare a 2-fold dilution series sample from 100 µg/mL to 3.1 µg/mL, and a calibration curve solution was prepared. 50 µL of the calibration curve solution or the mRNAlipid nanoparticles was mixed with 450 µL of methanol to prepare a measurement solution.

The absorbance of each measurement solution at 260 nm and 330 nm was measured using a UV plate reader (Multiskan Go, Thermo Fisher Scientific), the absorbance at 330 nm was subtracted from the absorbance at 260 nm, and the result was defined as the absorbance of each measurement solution. The total water phase mRNA concentration was calculated from the calibration curve using the absorbance of each sample measurement solution.

### (Quantification of mRNA concentration in outer water phase)

The concentration of the outer water phase nucleic acid was quantified by a standard addition method using a QuanT-iT RiboGreen RNA Assay Kit (Thermo Fisher Scientific). First, a 20× TE buffer included in the above kit was diluted with water, thereby obtaining a 1× TE buffer. TE represents Tris/EDTA (ethylenediaminetetraacetic acid). FLuc mRNA was diluted with a TE buffer such that the final concentration was 0 to 400 ng/mL to prepare a nucleic acid diluted series. 10 µL of mRNA lipid nanoparticles diluted 5-fold with TE buffer and 90 µL of a nucleic acid dilution series were mixed in a 96-well plate, 100 µL of a Ribogreen reagent diluted 200-fold with TE buffer was added to each well, and then fluorescence (excitation wavelength: 485 nm, fluorescence wavelength: 535 nm) was measured using a fluorescence plate reader (Infinite 200 Pro M nano +, TECAN). From the obtained results, the outer water phase mRNA concentration of each measurement solution was calculated according to the standard addition method.

### (Calculation of encapsulation rate)

By using the quantification results of the total mRNA concentration and the mRNA concentration in the outer water phase that were obtained through the above steps, the mRNA encapsulation rate of the mRNA lipid nanoparticles was calculated according to the following Equation. The results are shown in Table 1. mRNA encapsulation rate (%) = (total mRNA concentration-mRNA concentration in outer water phase)/total mRNA concentration × 100

The results are shown in Table 6.

**[Table 6]**

| Example | Ionizable lipid | Compound number | Particle size nm | PDI | Encapsulation rate % |
|---|---|---|---|---|---|
| Example 17 | Compound A | Compound 1 | 119 | 0.073 | 82.9 |
| Example 18 | Compound A | Compound 2 | 131 | 0.044 | 87.3 |
| Example 19 | Compound A | Compound 3 | 191 | 0.054 | 76.7 |
| Example 20 | Compound A | Compound 4 | 122 | 0.093 | 81.7 |
| Example 21 | Compound A | Compound 5 | 127 | 0.059 | 82.1 |
| Example 22 | Compound A | Compound 9 | 117 | 0.046 | 87.9 |
| Example 24 | Compound A | Compound 16 | 121 | 0.160 | 74.0 |
| Example 25 | Compound B | Compound 4 | 151 | 0.093 | 62.0 |
| Example 26 | Compound B | Compound 15 | 138 | 0.094 | 83.4 |
| Example 27 | Compound B | Compound 16 | 112 | 0.128 | 88.3 |

### <Luciferase expression measurement in vitro>

The luciferase expression measurement was performed in vitro.

### (Cells used for evaluation)

In an in vitro test using Hela cells, E-MEM (gibco), fetal bovine serum (FBS, gibco), Penicillin-streptomycin (gibco), and non-essential amino acid (NEAA, FUJIFILM Wako Pure Chemical Corporation) were mixed at a ratio of 88:10:1:1 and the mixture was used as a culture medium.

### (Quantification of luciferase expression level)

The luciferase expression level was measured according to the protocol of the ONE-Glo Luciferase Assay System (Promega). A dispersion liquid of nucleic acid lipid particles prepared in advance by pre-incubating hApoE (FUJIFILM Wako Pure Chemical Corporation) was added to the Hela cells such that the final concentration thereof was 80 ng/well as the mRNA concentration. After exposure for 24 hours under control of 37°C and 5% CO₂, a mixture of Luciferase Assay Buffer and Luciferase Assay Substrate was added thereto at 100 µL/well, and the mixture was allowed to stand at room temperature for 3 minutes to obtain a measurement solution. Each 50 µL of the measurement solution was transferred to another plate, and the amount of luminescence was measured with a plate reader (Envision, PerkinElmer). The results are shown in Table 7.

**[Table 7]**

| Example | Luciferase expression level (photon/sec) |
|---|---|
| Example 17 | 88,067 |
| Example 18 | 50,093 |
| Example 19 | 40,453 |
| Example 20 | 45,533 |
| Example 21 | 6,693 |
| Example 22 | 39,307 |
| Example 24 | 1,387 |
| Example 25 | 18,013 |
| Example 26 | 55,107 |
| Example 27 | 4,347 |

It was found that the nucleic acid lipid composition according to the embodiment of the present invention could confirm good expression of the luciferase and had excellent mRNA delivery ability.

## Claims

1. A lipid composition comprising:
a compound represented by Formula (1);
a lipid represented by Formula (2) or a salt of the lipid;
a neutral lipid;
a lipid having a nonionic hydrophilic polymer; and
a nucleic acid, 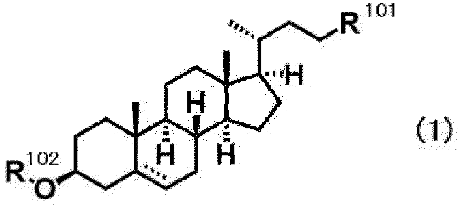 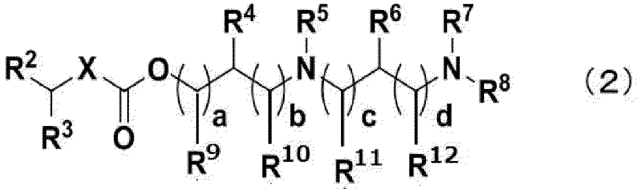
in Formula (1),
R¹⁰¹ represents 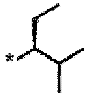 or 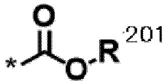
, * represents a bonding position, R²⁰¹ represents a chain-like hydrocarbon group having 7 to 18 carbon atoms, 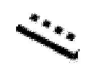
represents a single bond or a double bond,
R¹⁰² represents
(i) a hydrogen atom,
(ii) a hydrocarbon group having 1 to 4 carbon atoms, which may be substituted with one or more substituents selected from OH, SH, COOH, NR¹⁰⁶R¹⁰⁷, N(R¹⁰⁸)C(O)R¹⁰⁹, or CF₃,
(iii) C(O)R¹⁰³, or
(iv) C(O)NR¹⁰⁴R¹⁰⁵,
R¹⁰³ represents a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
R¹⁰⁴ and R¹⁰⁵ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃,
R¹⁰⁶, R¹⁰⁷, R¹⁰⁸, and R¹⁰⁹ each independently represent a hydrogen atom, or a hydrocarbon group having 1 to 4 carbon atoms, which may be substituted with one or more substituents selected from OH, SH, COOH, NR¹¹⁰R¹¹¹, N(R¹¹²)C(O)R¹¹³, or CF₃, and
R¹¹⁰, R¹¹¹, R¹¹², and R¹¹³ each independently represent a hydrogen atom or a hydrocarbon group having 1 to 4 carbon atoms, which may be substituted with one or more substituents selected from OH or SH,
in Formula (2), X represents -NR¹- or -O-,
R¹ represents a hydrogen atom, a hydrocarbon group having 6 to 24 carbon atoms, or a group represented by R²¹-L¹-R^{22_}, where R²¹ represents a hydrocarbon group having 1 to 24 carbon atoms, L¹ represents -O(CO)O-, -O(CO)-, -(CO)O-, -O-, or 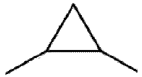
, and R²² represents a divalent hydrocarbon linking group having 1 to 18 carbon atoms,
Rand R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 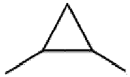
, and R³² represents a divalent hydrocarbon linking group having 1 to 18 carbon atoms,
R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
in any one or more pairs of groups among R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸, the groups may be linked to each other to form a 4- to 7-membered ring which may contain an O atom,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by - O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more.

2. The lipid composition according to claim 1,
wherein in Formula (1), R¹⁰² represents
(i) a hydrogen atom, or
(iv) C(O)NR¹⁰⁴R¹⁰⁵,
R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³, and
R¹¹⁰, R¹¹¹, R¹¹², and R¹¹³ each independently represent a hydrogen atom or a hydrocarbon group having 1 to 4 carbon atoms, which may be substituted with one or more OH's.

3. The lipid composition according to claim 1,
wherein R¹⁰¹ in Formula (1) represents 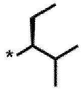 , and
R¹⁰² represents C(O)NR¹⁰⁴R¹⁰⁵,
R¹⁰⁴ and R¹⁰⁵ each independently represent a hydrogen atom or a hydrocarbon group having 1 to 4 carbon atoms, which may be substituted with one or more substituents selected from OH, NR¹¹⁰R¹¹¹, or N(R¹¹²)C(O)R¹¹³,
R¹¹⁰, R¹¹¹, R¹¹², and R¹¹³ each independently represent a hydrogen atom or a hydrocarbon group having 1 to 4 carbon atoms, which may be substituted with one or more OH's.

4. The lipid composition according to claim 1,
wherein R¹⁰¹ in Formula (1) represents 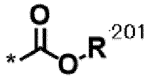
, R¹⁰² represents a hydrogen atom, and R²⁰¹ represents a chain-like hydrocarbon group having 7 to 13 carbon atoms.

5. The lipid composition according to any one of claims 1 to 4,
wherein the compound represented by Formula (1) is a compound represented by any of (A) to (M),

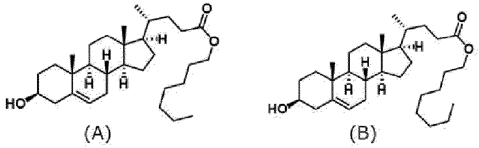

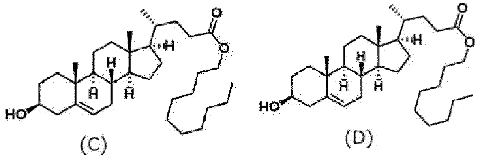

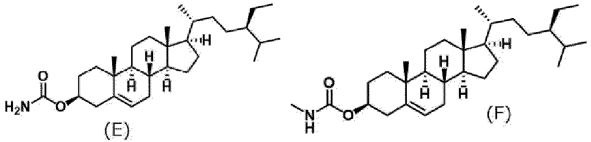

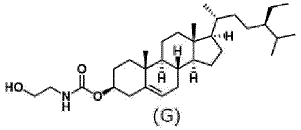

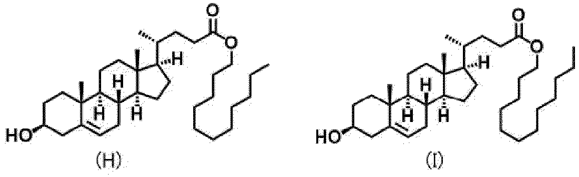

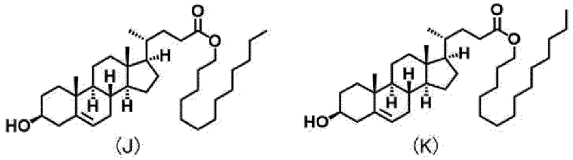

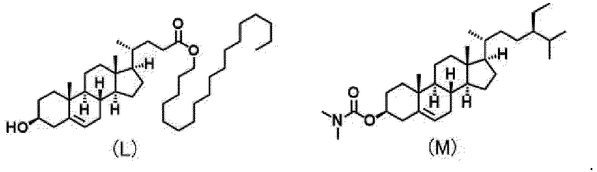

6. The lipid composition according to any one of claims 1 to 5,
wherein a content of the compound represented by Formula (1) is 10 mol% to 60 mol% with respect to total lipids.

7. The lipid composition according to any one of claims 1 to 6,
wherein a content of the lipid represented by Formula (2) or the salt of the lipid is 30 mol% to 70 mol% with respect to total lipids.

8. The lipid composition according to any one of claims 1 to 7,
wherein the neutral lipid is a zwitterionic lipid.

9. The lipid composition according to any one of claims 1 to 8,
wherein the neutral lipid is a phospholipid.

10. The lipid composition according to any one of claims 1 to 9,
wherein a content of the neutral lipid is 1 mol% to 30 mol% with respect to total lipids.

11. The lipid composition according to any one of claims 1 to 10,
wherein the lipid having the nonionic hydrophilic polymer contains an acyl group, and a carbon chain length of the acyl group is 8 to 26.

12. The lipid composition according to any one of claims 1 to 11,
wherein a content of the lipid having the nonionic hydrophilic polymer is 0.1 mol% to 10 mol% with respect to total lipids.

13. The lipid composition according to any one of claims 1 to 12,
wherein a weight ratio of lipids to the nucleic acid is 5 to 100.

14. The lipid composition according to any one of claims 1 to 13,
wherein the lipid composition is a composition for introducing a nucleic acid into a cell.

15. The lipid composition according to any one of claims 1 to 13,
wherein the lipid composition is a composition for nucleic acid delivery in vivo.

16. The lipid composition according to any one of claims 1 to 15,
wherein the lipid composition is a lipid particle.
